# EUROPEAN PATENT SPECIFICATION

(11) **EP 4 645 159 B1**
(45) Date of publication and mention of the grant of the patent: **25.11.2026**
(21) Application number: 24191186.6
(22) Date of filing: 26.07.2024
(51) Int. Cl.: G06M 1/06, G06M 1/08, G06M 1/14, G06M 3/04, G06M 3/12, A61M 15/00

(54) **AEROSOL ACTUATOR AND METERED DOSE INHALER**
AEROSOLAKTUATOR UND DOSIERINHALATOR
ACTIONNEUR D'AÉROSOL ET INHALATEUR DOSEUR

(30) Priority: 30.04.2024 CN 202410539957
(43) Date of publication of application: 05.11.2025
(73) Proprietor: Herchi Pharmaceutical (Shandong) Co., Ltd., Taian Shandong 271025 (CN)
(72) Inventor: WU, Mei, Taian Shandong 271025 (CN); LI, Huaidong, Taian Shandong 271025 (CN)
(74) Representative: Wang, Bo

(56) References cited:
- EP-B1- 1 047 467
- CN-U- 219 642 268
- US-A1- 2007 277 817
- US-A1- 2022 347 408

## Description

### TECHNICAL FIELD OF THE DISCLOSURE

The present disclosure relates to an aerosol actuator and a metered dose inhaler.

### BACKGROUND OF THE DISCLOSURE

Pressured metered dose inhalers (pMDls) can quantitatively spray drugs in a mist form, so that drug particles can go through the throat and directly act on the patient's lungs, achieving the purpose of efficient drug delivery. A pressured metered dose inhaler typically comprises an aerosol canister and an actuator. The aerosol canister comprises a canister body and a valve assembly. The canister body, with a drug contained in it, can move relative to the valve assembly. When pressed down to a certain distance, the canister body is opened, and a mist of drug particles is ejected from the canister body. Completing these actions requires the aerosol canister to coordinate with the actuator. Some actuators have a counting function, which allows the user to know the amount of a drug remaining in the aerosol canister and allows the user to prepare in advance, thereby avoiding a life-threatening situation caused by a lack of the drug.

CN219642268U discloses a full-counting driver that comprises a housing, a driving mechanism, and a transmission assembly. The housing is provided with a spray nozzle and a display port. The driving mechanism is arranged in the housing. The driving mechanism comprises a rotating shaft, a display wheel arranged on the outer wall of the rotating shaft, a driving tooth, a first driving gear, and a second driving gear. The driving tooth is arranged on the side wall on the display wheel side, and the first driving gear is arranged on the side wall on the other side of the display wheel; the second driving gear is arranged on one side of the outer wall of the first driving gear that is away from the driving tooth; one side of the outer wall of the display wheel that is close to the driving tooth is provided with a connection groove. The transmission assembly is arranged in the housing, and comprises a guide rod, a spring, and a hook. The guide rod is slidably arranged at the bottom of a base through the spring, the hook is fixedly arranged on the guide rod, and one end of the outer wall of the hook that is away from the guide rod is provided with a shifting tooth. When the transmission assembly of this device is reset, the driving mechanism is caused to reverse, impacting the accuracy of counting.

CN213852556U discloses an aerosol actuator that comprises a tube body, a counting disc, a one-way gear, an annular guide rail, and a shifting mechanism. The annular guide rail is arranged on the front of the tube body, the counting disc is arranged on the annular guide rail, and a circle of rack is arranged on the inner surface of the counting disc. The one-way gear is installed on the front of the tube body through a gear shaft, and a shifting tooth is arranged on the edge of the one-way gear. When the actuator is pressed, the shifting mechanism shifts the one-way gear to rotate by one tooth. When the one-way gear rotates by ten teeth, the shifting tooth comes into contact with the rack, rotating the counting disc by one measure. The counting disc of this actuator cannot count so accurately that it rotates once each time the one-way gear rotates by one tooth.

EP 1 047 467 B1 relates to an indicating device for indicating the number of metered dosages of a medicament that have been dispensed by, or remain in, a dispensing device.

### SUMMARY OF THE DISCLOSURE

In view of the above, one objective of the present disclosure is to provide an aerosol actuator according to appended claim 1 that enables the counting to be more accurate. Further, the aerosol actuator enables full utilization of a drug in the aerosol canister. Another objective of the present disclosure is to provide a metered dose inhaler according to appended claim 10. The present disclosure takes the technical solutions described hereafter to fulfill the above objectives.

The present disclosure provides an aerosol actuator, comprising a housing and a counting unit;
the housing comprises a housing body and a nozzle, the housing body being configured to communicate with the nozzle; the housing body being configured to accommodate the counting unit;
the counting unit is arranged in the housing body, and the counting unit comprises a gear fixing member, a counting gear, a driving element, and an elastic member;
a cavity is formed inside the gear fixing member;
the gear fixing member comprises a cover plate part, the cover plate part comprising a cover plate part body and a check pawl;
the counting gear is arranged in the cavity, and a surface of the counting gear that is close to the cover plate part body is provided with a limiting recess;
the check pawl is connected to the cover plate part body, and the check pawl is configured to coordinate with a meshing tooth of the counting gear so as to limit a rotation direction of the counting gear; the check pawl is provided with a limiting protrusion, the limiting protrusion being configured to coordinate with the limiting recess so as to limit a rotation direction of the counting gear;
the driving element is configured to move by an external force acting on it to thereby drive the counting gear to rotate;
the elastic member is configured to restore the driving element to its initial position when the external force is removed.

In the aerosol actuator according to the present disclosure, preferably, each of numerals displayed on the counting gear corresponds to one of the meshing teeth; and each of the meshing teeth corresponds to one of the limiting recesses.

In the aerosol actuator according to the present disclosure, preferably, the counting unit comprises a linkage gear; the number of the counting gears is at least two; two adjacent ones of the counting gears are linked through the linkage gear;
the gear fixing member further comprises a bracket part configured to coordinate with the cover plate part; the bracket part comprises a bracket part body and a bracket part stopper;
the counting gears comprise a lowest digit-position counting gear and a highest digit-position counting gear; the lowest digit-position counting gear is close to the cover plate part body, and the highest digit-position counting gear is close to the bracket part body;
the lowest digit-position counting gear is provided with a limiting recess; the check pawl is configured to coordinate with a meshing tooth of the lowest digit-position counting gear to thereby limit a rotation direction of the lowest digit-position counting gear;
the bracket part stopper is arranged on the bracket part body;
a surface of the highest digit-position counting gear that is close to the bracket part body is provided with a stopping part; the stopping part coordinates with the bracket part stopper so as to inhibit the highest digit-position counting gear from rotating in a counting direction when the highest digit-position counting gear counts down to 0.

In the aerosol actuator according to the present disclosure, preferably, the linkage gear is arranged between two adjacent ones of the counting gears; one side of the lowest digit-position counting gear that is close to the linkage gear is provided with a transmission tooth, and one side of the lowest digit-position counting gear that is close to the cover plate part body is provided with a meshing tooth; both sides of another low digit-position counting gear are provided with a transmission tooth and a meshing tooth, respectively; one side of the highest digit-position counting gear that is close to the linkage gear is provided with a meshing tooth;
the linkage gear is configured to be capable of engaging with the meshing tooth of one of two counting gears adjacent to it that is a high digit-position counting gear; the linkage gear and the counting gear are configured to function such that when a low digit-position counting gear counts down to 0 and then counts again, a transmission tooth of the low digit-position counting gear drives the linkage gear to rotate, and the linkage gear drives the high digit-position counting gear to rotate and count once.

In the aerosol actuator according to the present disclosure, preferably the gear fixing member comprises a first fixing shaft and a second fixing shaft; the first fixing shaft and the second fixing shaft are arranged in the cavity, the first fixing shaft is positioned higher than the second fixing shaft; the first fixing shaft is configured to fix the counting gear, and the second fixing shaft is configured to fix the linkage gear.

In the aerosol actuator according to the present disclosure, preferably, the driving element comprises a main body part and a driving pawl;
the main body part is provided with a pressing part; the pressing part is configured to receive an external force to cause the main body part to move;
the driving pawl is connected to the main body part and configured to drive, through a meshing tooth, the counting gear to rotate;
the driving pawl is provided with a deflection bending part; the deflection bending part is configured such that when the counting gear rotates normally, the deflection bending part does not affect the driving pawl in driving the counting gear to rotate, and when the counting gear is locked and an external force acts on the pressing part, the deflection bending part elastically deforms and causes the driving element to move.

In the aerosol actuator according to the present disclosure, preferably, the driving element further comprises a connection part, and the gear fixing member is provided with a fixing part;
the elastic member is sleeved around the connection part, one end of the elastic member abuts against the main body part, and the other end of the elastic member abuts against the fixing part;
the fixing part is provided with a through hole, and at least part of the connection part penetrates the through hole.

In the aerosol actuator according to the present disclosure, preferably, the main body part is provided with a first normal limiting structure and a first transverse limiting structure, and the gear fixing member is provided with a second normal limiting structure and a second transverse limiting structure;
the first normal limiting structure coordinates with the second normal limiting structure to thereby limit a normal movement path of the driving element; the first transverse limiting structure coordinates with the second transverse limiting structure to thereby limit a transverse movement path of the driving element;
a lower part of the connection part is provided with a longitudinal limiting structure configured to limit a longitudinal displacement of the driving element.

In the aerosol actuator according to the present disclosure, preferably, the aerosol actuator further comprises a dust cover;
a liquid inlet passage is arranged in the housing body and provided with a spray hole; the liquid inlet passage is configured to communicate with the nozzle through the spray hole;
the dust cover is sleeved around the nozzle.

Another aspect of the present disclosure is to provide a metered dose inhaler, comprising an aerosol canister and an aerosol actuator described above; at least part of the aerosol canister is configured to be accommodated in the housing body, and the liquid inlet passage is configured to communicate with a liquid outlet pipe of the aerosol canister.

The aerosol actuator of the present disclosure has a double-check structure capable of inhibiting the driving element from driving the counting gear to rotate in a direction opposite the counting direction when the driving element is reset; the configuration of the limiting protrusion and the limiting recess can also play a position-fixing role to inhibit the counting gear from rotating and counting due to accidental touch by users. This can make the counting more accurate. According to a preferred technical solution of the present disclosure, the driving element is provided with a deflection bending part. In this way, after the counting gear counts down to 0, the driving element can still move, which enables full use of a drug remaining in the aerosol canister.

### BRIEF SUMMARY OF THE DRAWINGS

FIG. 1 is an exploded diagram of an aerosol actuator of the present disclosure.
FIG. 2 is a schematic diagram showing a partial configuration of the housing of the aerosol actuator shown in FIG. 1.
FIG. 3 is a schematic diagram showing the counting unit of the aerosol actuator shown in FIG. 1.
FIG. 4 is an exploded diagram of the counting unit of the aerosol actuator shown in FIG. 1.
FIG. 5 is a schematic diagram showing a partial configuration of the cover plate part of the counting unit shown in FIG. 3.
FIG. 6 is a schematic diagram showing a partial configuration of the unit-digit gear of the counting unit shown in FIG. 3.
FIG. 7 is a schematic diagram showing a partial configuration of the driving element of the counting unit shown in FIG. 3.
FIG. 8 is a schematic diagram showing the configuration of the counting unit shown in FIG. 3 when viewed from the z-axis direction.
FIG. 9 is an exploded diagram of a metered dose inhaler according to the present disclosure.
FIG. 10 is a schematic diagram showing an assembling configuration of an aerosol canister and an aerosol actuator.
The reference numerals of the components are as follows:
110-cover plate part; 111-first connection sleeve; 112-second connection sleeve; 113-check pawl; 114-limiting protrusion; 115-second normal limiting structure; 116-fixing part; 117-cover plate part body; 120-bracket part; 131-limiting block; 132-second transverse limiting structure; 133-bracket part body; 134-top of the bracket part; 140-display window; 151-unit-digit gear; 1511-unit-digit gear meshing tooth; 1512-limiting recess; 1513-unit-digit gear transmission tooth; 152-ten-digit gear; 1521-transmission tooth of the ten-digit gear; 153-hundred-digit gear; 1531-stopping part; 161-first linkage gear; 162-second linkage gear; 171-first fixing shaft; 172-second fixing shaft; 181-first positioning post; 182-second positioning post; 183-third positioning post; 184-fourth positioning post; 191-first buckle; 192-second buckle; 2-driving element; 210-main body part; 211-pressing part; 212-first normal limiting structure; 213-first transverse limiting structure; 220-driving pawl; 221-deflection bending part; 230-connection part; 231-longitudinal limiting structure; 3-elastic member; 4-housing; 410-housing body; 411-liquid inlet passage; 412-spray hole; 413-rear wall; 414-observation window; 420-nozzle; 5-counting unit; 6-dust cover; 7-aerosol canister; 710-liquid outlet pipe.

### DETAIL DESCRIPTION OF THE DISCLOSURE

The present disclosure will be further explained by way of specific examples, but the protection scope of the present disclosure is not limited thereto.

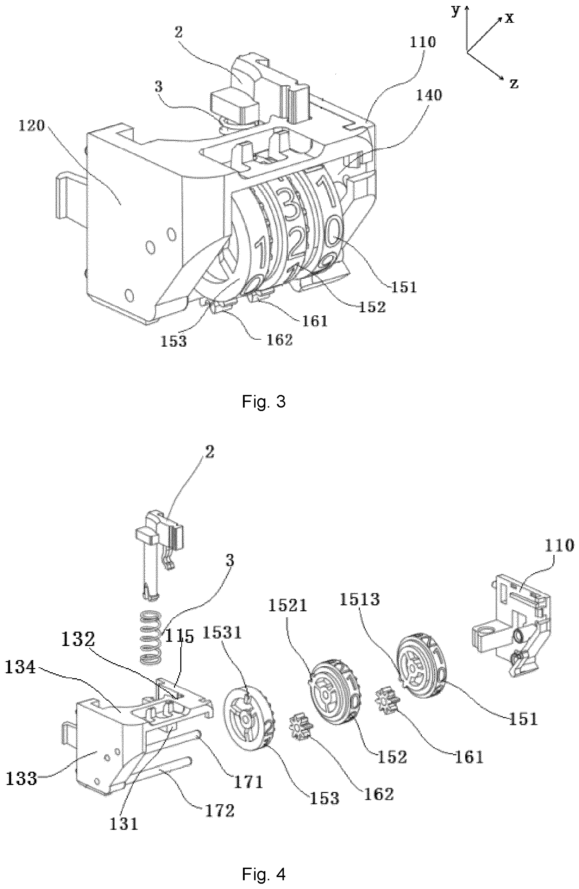

In the present disclosure, the transverse direction refers to the x direction shown in FIG. 3; the longitudinal direction refers to the y direction shown in FIG. 3; and the normal direction refers to the z direction shown in FIG. 3.

### <Aerosol Actuator>

The aerosol actuator of the present disclosure comprises a housing and a counting unit. In some embodiments, it further comprises a dust cover.

### Housing and dust cover

The housing of the present disclosure comprises a housing body and a nozzle. The housing body communicates with the nozzle. The housing body and the nozzle can be arranged substantially in an "L" shape.

The housing body is configured to accommodate an aerosol canister and the counting unit. The counting unit can be installed into the housing body through a positioning post, a positioning hole, and a connection member such as a buckle and a slot. This is convenient for accurately fixing the position of the counting unit. The housing body has a rear wall. The rear wall is arranged at a position far away from the nozzle. Part of the rear wall can be a detachable structure. The detachable structure can be connected to the other part of the housing body by a snap-fit joint. This is convenient for installing the counting unit into the housing body. The rear wall can be provided with an observation window. The projection of the observation window on the plane where a display window is located falls within the range of the display window. The observation window is configured to display the number of times that a drug remaining in the aerosol canister is available. The observation window can be arranged on the detachable part of the rear wall.

A liquid inlet passage is arranged in the housing body. The liquid inlet passage is configured to communicate with a liquid outlet pipe of the aerosol canister. The liquid inlet passage is provided with a spray hole. The liquid inlet passage communicates with the nozzle through the spray hole.

The dust cover is sleeved around the nozzle to prevent the nozzle from being contaminated.

### Counting unit

The counting unit of the present disclosure comprises a gear fixing member, a counting gear, a driving element, and an elastic member. In some embodiments, it further comprises a linkage gear. The following is a detailed description.

### Gear fixing member

A cavity is formed inside the gear fixing member of the present disclosure. The cavity is configured to accommodate the counting gear. In some embodiments, the gear fixing member has a cover plate part and a bracket part. The cover plate part comprises a cover plate part body. The bracket part comprises a bracket part body and a top. The cover plate part body and the bracket part body are configured to oppose each other. Part of the cover plate part body, part of the bracket part body, and part of the top of the bracket part constitute the cavity. The cavity can be provided with a display window. The display window is configured to display numerals on the counting gear. The bracket part body and the top of the bracket part can constitute an integrated structure. The cover plate part is connected to the bracket part through a connector, for example, by a snap-fit joint.

The cover plate part further comprises a check pawl. The check pawl is arranged on the cover plate part body. The check pawl is configured to coordinate with the meshing tooth of the counting gear so as to limit a rotation direction of the counting gear. The check pawl can engage with the meshing tooth of the counting gear, thereby inhibiting the counting gear from rotating in a direction opposite the counting direction. When there is more than one counting gear, the check pawl engages with the meshing tooth of the lowest digit-position counting gear (for example, the unit-digit gear). In some embodiments, one end of the check pawl is connected to the cover plate part body, and the other end of the check pawl is a free end. The free end of the check pawl engages with the counting gear.

The check pawl is provided with a limiting protrusion. The limiting protrusion is configured to coordinate with a limiting recess of the counting gear (the lowest digit-position counting gear, such as the unit-digit gear) so as to limit a rotation direction of the counting gear. The limiting protrusion is arranged at the free end of the check pawl. Specifically, the limiting protrusion is arranged on a surface of the free end that is close to the counting gear. The limiting protrusion can be a cylinder or can be formed by stacking multiple cylinders. When the limiting protrusion is formed by stacking multiple cylinders, the multiple cylinders are stacked in descending order of diameter, with the cylinder having the largest diameter being closest to the check pawl. In this way, the limiting protrusion and the check pawl can take a double-limiting effect and thereby make the counting more accurate.

The gear fixing member comprises a fixing shaft, and the fixing shaft is located in the cavity of the gear fixing member. The fixing shaft is configured to fix a gear. One end of the fixing shaft is fixed on the bracket part body, and the other end thereof can be fixed on the cover plate part. In some embodiments, the cover plate part further comprises a connection sleeve. The connection sleeve is arranged on the cover plate part body. The connection sleeve is arranged on the inner surface of the cover plate part body. The other end of the fixing shaft extends into the connection sleeve, thereby connecting the fixing shaft to the cover plate part. This is convenient for installing a gear on the fixing shaft.

In some embodiments, the fixing shaft comprises a first fixing shaft and a second fixing shaft. The first fixing shaft is positioned higher than the second fixing shaft. The first fixing shaft is configured to fix the counting gear. The second fixing shaft is configured to fix the linkage gear.

The bracket part further comprises a bracket part stopper. The bracket part stopper can be arranged on the bracket part body. The bracket part stopper is configured to coordinate with the stopping part such that it inhibits the counting gear (the counting gear provided with the stopping part) from continuing to count when the counting gear counts down to 0. When there is more than one counting gear, the bracket part stopper inhibits the highest digit-position counting gear (e.g., the hundred-digit gear) from continuing to count. The bracket part stopper can be of a protruded structure.

The gear fixing member can be provided with a limiting block. The limiting block can be arranged at the top of the bracket part. The limiting block is configured to limit the position of the counting gear.

The gear fixing member can be provided with a fixing part. The fixing part can abut against the elastic member. The fixing part is provided with a through hole. At least part of the connection part of the driving element is configured to penetrate the through hole. The fixing part can be arranged on the cover plate part body.

The gear fixing member can be provided with a second transverse limiting structure. The second transverse limiting structure is configured to coordinate with the first transverse limiting structure so as to limit a transverse movement path of the driving element. The second transverse limiting structure can be a dovetail mortise. The second transverse limiting structure can be arranged at the top of the bracket part.

The gear fixing member can be provided with a second normal limiting structure. The second normal limiting structure is configured to coordinate with the first normal limiting structure so as to limit a normal motion path of the driving element. The second normal limiting structure can be a dovetail tenon. In some embodiments, the second normal limiting structure is arranged on the cover plate part body. In other embodiments, one end of the second normal limiting structure is connected to the top of the bracket part and extends downwards along the cover plate part body.

### Counting gear and linkage gear

The counting gear is arranged in the cavity. The counting gear can be sleeved around the first fixing shaft. The surface of the counting gear is provided with a limiting recess. The limiting recess coordinates with the limiting protrusion (which can be located on the check pawl) on the gear fixing member so as to inhibit the counting gear from rotating in a direction opposite the counting direction. The limiting recess is arranged on a surface of the counting gear that is close to the cover plate part body. The limiting recess and the meshing tooth are arranged on the same surface. When there is more than one counting gear, the limiting recess is arranged on the lowest digit-position counting gear (e.g., the unit-digit gear).

Each of the numerals displayed on the counting gear corresponds to one of the meshing teeth. The driving element is capable of engaging with the meshing tooth of the lowest digit-position counting gear so as to drive the counting gear to count. There can be more than one limiting recess. Each of the meshing teeth corresponds to one of the limiting recesses.

There can be more than one counting gear. The two adjacent ones of the counting gears are linked by a linkage gear. The linkage gear is arranged between the two counting gears to which the linkage gear is linked. The lowest digit-position counting gear is close to the cover plate part body, and the highest digit-position counting gear is close to the bracket part body. The digit positions of the counting gears increase in sequence from the cover plate part body to the bracket part body. The linkage gear can be sleeved around the second fixing shaft.

One side of the lowest digit-position counting gear that is close to the linkage gear is provided with a transmission tooth, and one side of the lowest digit-position counting gear that is close to the cover plate part body is provided with a meshing tooth; both sides of another low digit-position counting gear are provided with a transmission tooth and a meshing tooth, respectively; one side of the highest digit-position counting gear that is close to the linkage gear is provided with a meshing tooth. The transmission tooth can be two half teeth. The linkage gear engages with the meshing tooth of one of two counting gears adjacent to it that is a high digit-position counting gear; when a low digit-position counting gear counts down to 0 and then counts again, the transmission tooth of the low digit-position counting gear drives the linkage gear to rotate, and the linkage gear drives the high digit-position counting gear to rotate and count once.

The counting gear is provided with a stopping part. The stopping part is arranged on a surface of the counting gear that is close to the bracket part body. When there is more than one counting gear, the stopping part is arranged on the highest digit-position counting gear. The stopping part can be of a protruded structure. When the counting gear counts down to 0, the stopping part and the bracket part stopper abut against each other, thereby inhibiting the counting gear from continuing to rotate in the counting direction.

In one embodiment, according to the present disclosure, the counting gear comprises a unit-digit gear, a ten-digit gear, and a hundred-digit gear. The linkage gear comprises a first linkage gear and a second linkage gear.

The unit-digit gear, the ten-digit gear, and the hundred-digit gear are sequentially arranged on the first fixing shaft. The unit-digit gear is close to the cover plate part body. The hundred-digit gear is close to the bracket part body. The ten-digit gear is arranged between the unit-digit gear and the hundred-digit gear.

The first linkage gear and the second linkage gear are arranged on the second fixing shaft. The first linkage gear is arranged between the unit-digit gear and the ten-digit gear and configured to link the unit-digit gear and the ten-digit gear. The second linkage gear is arranged between the ten-digit gear and the hundred-digit gear and configured to link the ten-digit gear and the hundred-digit gear.

A surface of the unit-digit gear that is close to the cover plate body is provided with a unit-digit meshing tooth and a limiting recess. A surface of the unit-digit gear that is close to the ten-digit gear is provided with a unit-digit transmission tooth.

A surface of the ten-digit gear that is close to the unit-digit gear is provided with a ten-digit meshing tooth. A surface of the ten-digit gear that is close to the hundred-digit gear is provided with a ten-digit transmission tooth.

A surface of the hundred-digit gear that is close to the ten-digit gear is provided with a hundred-digit meshing tooth. A surface of the hundred-digit gear that is close to the bracket part body is provided with a stopping part.

### Driving element and elastic member

The driving element is configured to move by an external force acting on it to thereby drive the counting gear to rotate. The driving element comprises a main body part and a driving pawl.

The main body part is provided with a pressing part. The pressing part is configured to receive an external force to cause the main body part to move.

The main body part is provided with a first normal limiting structure. The first normal limiting structure is configured to coordinate with the second normal limiting structure so as to limit a normal movement path of the driving element. The first normal limiting structure can be a dovetail mortise. The first normal limiting structure can be arranged on a side wall of the main body part that is close to the cover plate part body.

The main body part is provided with a first transverse limiting structure. The first transverse limiting structure is configured to coordinate with the second transverse limiting structure so as to limit a transverse movement path of the driving element. The first transverse limiting structure can be a dovetail tenon. The first transverse limiting structure can be arranged on a side wall of the main body part that is close to the counting gear.

The driving pawl is connected to the main body part. The driving pawl is configured to drive, through the meshing tooth, the counting gear (the lowest digit-position counting gear) to rotate. The main body part moves downwards under external pressure and thereby drives the driving pawl to move downwards. The driving pawl engages with the meshing tooth and thereby drives the counting gear to rotate in the counting direction. The driving pawl engages with the meshing tooth of the lowest digit-position counting gear (the unit-digit gear) and thereby drives the lowest digit-position counting gear (the unit-digit gear) to rotate in the counting direction. In some embodiments, one end of the driving pawl is connected to the lower surface of the main body part, and the other end thereof is a free end capable of engaging with the meshing tooth of the counting gear.

The driving pawl is provided with a deflection bending part. The deflection bending part is configured such that when the counting gear rotates normally, the deflection bending part does not affect the driving pawl in driving the counting gear to rotate, and when the counting gear is locked and an external force acts on the pressing part, the deflection bending part elastically deforms and causes the driving element to move. The deflection bending part can be arranged in the middle of the driving pawl. The deflection bending part can be realized through the design of materials and bending structures.

The elastic member is configured to restore the driving element to its initial position when the external force is removed. The driving element is further provided with a connection part.

One end of the connection part is connected to the main body part. Specifically, it is connected to the lower surface of the main body part. The other end of the connection part is a free end. Part of the connection part is located in the through hole of the fixing part of the gear fixing member. The free end of the connection part can be provided with a longitudinal limiting structure. The longitudinal limiting structure limits a longitudinal displacement of the driving element. The longitudinal limiting structure can be a cotter pin. The projection of the connection part on the plane where the lower surface of the main body part is located falls within the range of the lower surface of the main body part.

The elastic member is sleeved around the outer circumference of the connection part. One end of the elastic member abuts against the main body part, and the other end of the elastic member abuts against the fixing part. The elastic member can be a spring.

### <Metered Dose Inhaler>

The metered dose inhaler of the present disclosure comprises an aerosol canister and an aerosol actuator. The aerosol actuator has been described hereinbefore, and no description thereof is necessary here.

At least part of the aerosol canister is accommodated in the housing body. The liquid outlet pipe of the aerosol canister is arranged in the liquid inlet passage. The edge of the cover body of the aerosol canister is in contact with the pressing part of the driving element

### Example 1

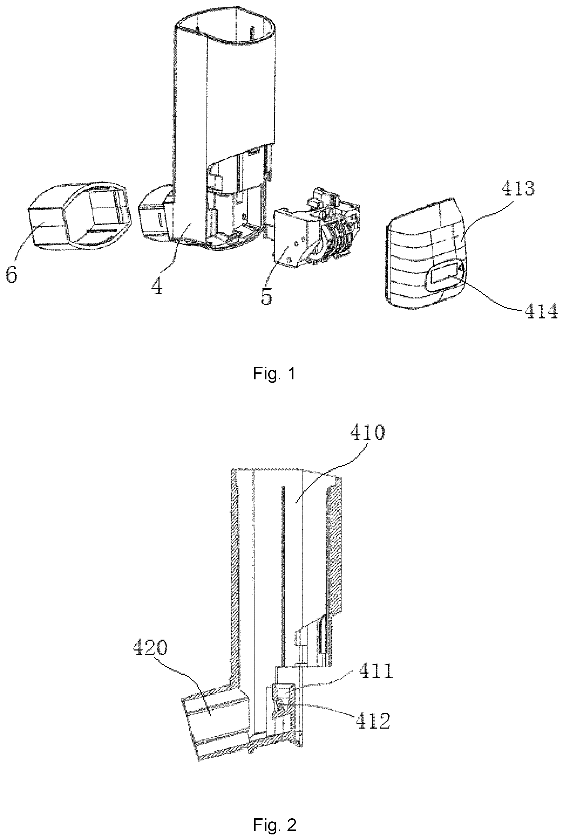

As shown in FIG. 1, the aerosol actuator of the present disclosure comprises a housing 4, a counting unit 5, and a dust cover 6.

As shown in FIG. 2, the housing 4 comprises a housing body 410 and a nozzle 420. The housing body 410 communicates with the nozzle 420. The housing body 410 and the nozzle 420 can be arranged substantially in an "L" shape. The housing body 410 is configured to accommodate an aerosol canister and the counting unit 5. A liquid inlet passage 411 is arranged in the housing body 410. The liquid inlet passage 411 is configured to communicate with a liquid outlet pipe of the aerosol canister. The liquid inlet passage 411 is provided with a spray hole 412. The liquid inlet passage 411 communicates with the nozzle 420 through the spray hole.

As shown in FIG. 1, the dust cover 6 is sleeved around the nozzle 420. The housing body 410 has a rear wall 413 that is arranged away from the nozzle 420. Part of the rear wall 413 is a detachable structure. This facilitates the installation of the counting unit 5 into the housing body 410. The detachable structure can be connected to the other part of the rear wall 413 by a snap-fit joint. The rear wall 413 is provided with an observation window 414 for displaying the number of times that a drug remaining in the aerosol canister is available. The observation window 414 can be arranged on the detachable part of the rear wall 413.

As shown in FIGS. 3 and 4, the counting unit 5 comprises a gear fixing member, a counting gear, a linkage gear, a driving element 2, and an elastic member 3.

The gear fixing member has a cover plate part 110 and a bracket part 120. The cover plate part 110 comprises a cover plate part body 117. The bracket part 120 has a bracket part body 133 and a top 134. The cover plate part body 117 and the bracket part body 133 are configured to oppose each other. Part of the cover plate part body 117, part of the bracket part body 133, and part of the top 134 of the bracket part constitute a cavity. The cavity is configured to accommodate the counting gear and the linkage gear. The cavity has a display window 140 for displaying the numerals on the counting gear.

As shown in FIG. 4, a first fixing shaft 171 and a second fixing shaft 172 are arranged in the cavity. The first fixing shaft 171 is positioned higher than the second fixing shaft 172. One end of the first fixing shaft 171 is fixed to the bracket part body 133, and the other end of the first fixing shaft 171 can be fixed to the cover plate part 110 through a connection member. One end of the second fixing shaft 172 is fixed to the bracket part body 133, and the other end of the second fixing shaft 172 can be fixed to the cover plate part 110 through a connection member.

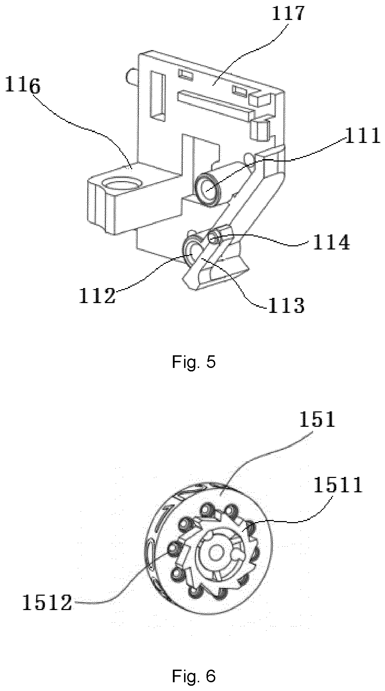

In some embodiments, the bracket part body 133 and the top 134 can constitute an integrated structure, and the cover plate part body 117 is connected to the top 134 through a connector, for example, by a snap-fit joint. As shown in FIG. 5, the cover plate part 110 further comprises a first connection sleeve 111 and a second connection sleeve 112. The first connection sleeve 111 and the second connection sleeve 112 are arranged on the inner surface of the cover plate part body 117. The other end of the first fixing shaft 171 extends into the first connection sleeve 111, thereby connecting the first fixing shaft 171 to the cover plate part 110. The other end of the second fixing shaft 172 extends into the second connection sleeve 112, thereby connecting the second fixing shaft 172 to the cover plate part 110. Thus, the counting gear and the linkage gear are sleeved around the first fixing shaft 171 and the second fixing shaft 172, respectively.

As shown in FIGS. 3 and 4, the counting gear comprises a unit-digit gear 151, a ten-digit gear 152, and a hundred-digit gear 153. The unit-digit gear 151, the ten-digit gear 152, and the hundred-digit gear 153 are all sleeved around the first fixing shaft 171. The unit-digit gear 151 is close to the cover plate part body 117, the ten-digit gear 152 is arranged between the unit-digit gear 151 and the hundred-digit gear 153, and the hundred-digit gear 153 is close to the bracket part body 133. The top 134 of the bracket part can be provided with a limiting block 131. The limiting block 131 is configured to limit the position of the counting gear.

The linkage gear comprises a first linkage gear 161 and a second linkage gear 162. The first linkage gear 161 and the second linkage gear 162 are sleeved around the second fixing shaft 172. The first linkage gear 161 is arranged between the unit-digit gear 151 and the ten-digit gear 152 and configured to link the unit-digit gear 151 and the ten-digit gear 152. The second linkage gear 162 is arranged between the ten-digit gear 152 and the hundred-digit gear 153 and configured to link the ten-digit gear 152 and the hundred-digit gear 153.

As shown in FIG. 6, a surface of the unit-digit gear 151 that is close to the cover plate part body 117 is provided with a unit-digit gear meshing tooth 1511. There can be more than one unit-digit gear meshing tooth 1511. Each of the numerals displayed on the unit-digit gear 151 corresponds to one of the unit-digit gear meshing teeth 1511. The driving element 2 drives, through the unit-digit gear meshing tooth 1511, the unit-digit gear 151 to rotate. Each time the unit-digit gear 151 is rotated, the numerals displayed on it decrease by one.

As shown in FIG. 4, a surface of the ten-digit gear 152 that is close to the unit-digit gear 151 is provided with a ten-digit gear meshing tooth (not shown). There can be more than one ten-digit gear meshing tooth. Each of the numerals displayed on the ten-digit gear 152 correspond to one of the ten-digit gear meshing teeth. The ten-digit gear meshing tooth engages with the first linkage gear 161.

A surface of the unit-digit gear 151 that is away from the cover plate part body 117 is provided with a unit-digit gear transmission tooth 1513. When the unit-digit gear 151 counts down to 0 and then counts again, the unit-digit gear transmission tooth 1513 drives the first linkage gear 161 to rotate, and the first linkage gear 161 drives the ten-digit gear 152 to rotate and count once.

A surface of the hundred-digit gear 153 that is close to the ten-digit gear 152 is provided with a hundred-digit gear meshing tooth (not shown). There can be more than one hundred-digit gear meshing tooth. Each of the numerals displayed on the hundred-digit gear 153 corresponds to one of the hundred-digit gear meshing teeth. The hundred-digit gear meshing tooth engages with the second linkage gear 162.

A surface of the ten-digit gear 152 that is close to the hundred-digit gear 153 is provided with a ten-digit gear transmission tooth 1521. When the ten-digit gear 152 counts down to 0 and then counts again, the ten-digit gear transmission tooth 1521 drives the second linkage gear 162 to rotate, and the second linkage gear 162 drives the hundred-digit gear 153 to rotate and count once.

A surface of the hundred-digit gear 153 that is close to the bracket part body 133 is provided with a stopping part 1531. The stopping part 1531 can be a protruded structure. A bracket part stopper (not shown) is arranged on the inner surface of the bracket part body 133. The bracket part stopper can be a boss. When the hundred-digit gear 153 counts down to 0, the stopping part 1531 abuts against the bracket part stopper, thereby inhibiting the hundred-digit gear 153 from continuing to rotate in the counting direction. When both the unit-digit gear 151 and the ten-digit gear 152 count down to 0, two adjacent gears of the unit-digit gear 151, the first linkage gear 161, the ten-digit gear 152, the second linkage gear 162, and the hundred-digit gear 153 engage with each other. Since the hundred-digit gear 153 cannot continue to rotate in the counting direction, the unit-digit gear 151 and the ten-digit gear 152 cannot continue to rotate in the counting direction either, thereby locking the counting gear. This avoids overcounting and thereby makes the counting more accurate.

As shown in FIG. 5, the cover plate part 110 further comprises a check pawl 113. One end of the check pawl 113 is connected to the cover plate part body 117, and the other end of the check pawl 113 is a free end. The free end of the check pawl 113 engages with the unit-digit gear meshing tooth 1511, thereby inhibiting the unit-digit gear 151 from rotating in a direction opposite the counting direction. A surface of the free end of the check pawl 113 that is close to the unit-digit gear 151 is provided with a limiting protrusion 114. The limiting protrusions 114 can be a cylinder or can be formed by stacking multiple cylinders. When the limiting protrusion 114 is formed by stacking multiple cylinders, the multiple cylinders are stacked in descending order of diameter, with the cylinder having the largest diameter being closest to the check pawl 113.

As shown in FIG. 6, a surface of the unit-digit gear 151 that is close to the cover plate part body 117 is provided with a limiting recess 1512 configured to coordinate with the limiting protrusion 114. There can be more than one limiting recess 1512. Each of the unit-digit gear meshing teeth 1511 corresponds to one of the limiting recesses 1512. The limiting protrusion 114 coordinates with the limiting recess 1512 so as to inhibit the unit-digit gear 151 from rotating in a direction opposite the counting direction. The limiting protrusion 114 together with the limiting recess 1512 and the check pawl 113 takes a double-check effect, inhibiting the driving element 2 from driving the unit-digit gear 151 to rotate in a direction opposite the counting direction when the driving element 2 is reset. Meanwhile, the limiting recess 1512 and the limiting protrusion 114 also take a position-fixing function, inhibiting the counting gear from rotating due to accidental touch and thereby making the counting more accurate.

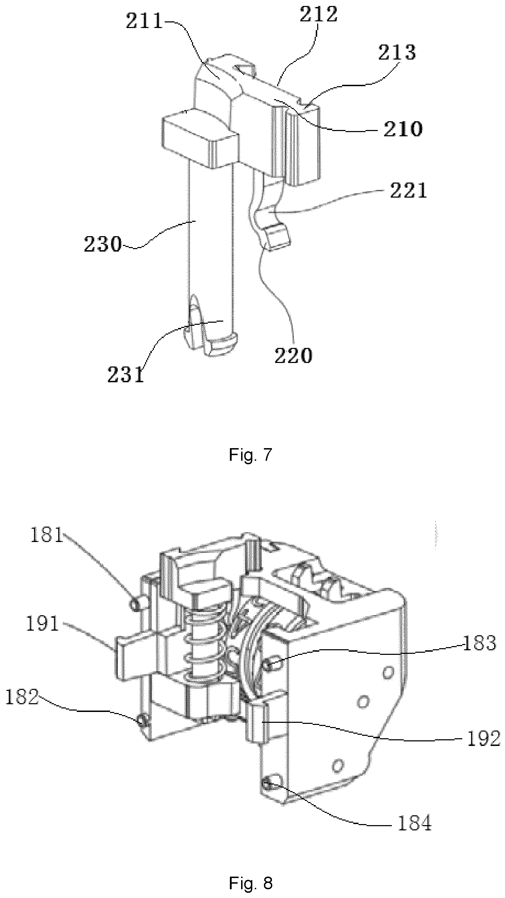

As shown in FIG. 7, the driving element 2 comprises a main body part 210, a driving pawl 220, and a connection part 230.

The upper surface of the main body part 210 is provided with a pressing part 211. An external force acts on the pressing part 211 to move the main body part 210. A side wall of the main body part 210 that is close to the cover plate part body 117 is provided with a first normal limiting structure 212. The first normal limiting structure 212 can be a dovetail mortise. As shown in FIG. 4, the second normal limiting structure 115 can be arranged on the inner surface of the cover plate part body 117. In other embodiments, one end of the second normal limiting structure 115 can be connected to the top 134 of the bracket part and extend downward along the cover plate part body 117. The second normal limiting structure 115 can be a dovetail tenon. The first normal limiting structure 212 coordinates with the second normal limiting structure 115 so as to limit a normal movement path of the driving element 2.

As shown in FIGS. 4 and 7, a side wall of the main body part 210 that is close to the counting gear is provided with a first transverse limiting structure 213. The first transverse limiting structure 213 can be a dovetail tenon. The top 134 of the bracket part is provided with a second transverse limiting structure 132. The second transverse limiting structure 132 can be a dovetail mortise. The first transverse limiting structure 213 and the second transverse limiting structure 132 coordinate with each other so as to limit a transverse movement path of the driving element 2.

One end of the driving pawl 220 is connected to the lower surface of the main body part 210, and the other end of the driving pawl 220 is a free end. The free end of the driving pawl 220 can engage with the unit-digit gear meshing tooth 1511 so as to drive the unit-digit gear 151 to rotate in the counting direction. The driving pawl 220 is provided with a deflection bending part 221. The deflection bending part 221 can be arranged in the middle of the driving pawl 220. When the counting gear rotates normally, the deflection bending part 221 does not affect the driving pawl 220 in driving the counting gear to rotate; when the counting gear is locked (in this embodiment, at this point the unit-digit gear 151, the ten-digit gear 152, and the hundred-digit gear 153 all count down to 0) and an external force acts on the pressing part 211, the deflection bending part elastically deforms and causes the driving element 2 to move. In this way, the driving element 2 does not inhibit the aerosol canister from moving relative to the aerosol canister valve stem. And when the counting gear counts down to 0, the aerosol canister can still move relative to the aerosol canister valve stem to a position where the aerosol canister can be opened, so that a drug remaining in the aerosol canister can be ejected, making it possible to make full use of the drug. The deflection bending part 221 can be realized through the design of materials and bending structures.

As shown in FIG. 7, one end of the connection part 230 is connected to the lower surface of the main body part 210, and the other end of the connection part 230 is a free end. The projection of the connection part 230 on the plane where the lower surface of the main body part 210 is located falls within the range of the lower surface of the main body part 210.

As shown in FIG. 5, the cover plate part 110 further comprises a fixing part 116. The fixing part 116 is arranged on the inner surface of the cover plate part body 110. The fixing part 116 is provided with a through hole. Part of the connection part 230 penetrates the through hole. The free end of the connection part 230 penetrates the through hole. The free end of the connection part 230 is provided with a longitudinal limiting structure 231. The longitudinal limiting structure 231 can be a cotter pin. The longitudinal limiting structure 231 limits a longitudinal displacement of the driving element 2.

The elastic member 3 is sleeved on the outer periphery of the connection part 230. One end of the elastic member 3 abuts against the lower surface of the main body part 210, and the other end of the elastic member 3 abuts against the fixing part 116. The elastic member 3 is configured to restore the driving element 2 to its initial position when the external force is removed. The elastic member 3 can be a spring.

When an external force is applied to the pressing part 211, the driving element 2 moves downward, the elastic member 3 contracts, and the driving pawl 220 engages with the unit-digit gear meshing tooth 1511 and drives the unit-digit gear 151 to rotate in the counting direction and count once. When the external force acting on the pressing part 211 is removed, the elastic member 3 is restored to the initial state, and the driving element 2 is restored to the initial position.

As shown in FIG. 8, the upper and lower parts of a surface of the cover plate part body 117 that is away from the rear wall 413 are provided with a first positioning post 181 and a second positioning post 182, respectively, and the middle part of the surface is provided with a first buckle 191. The upper and lower parts of a surface of the bracket part body 133 that is away from the rear wall 413 are provided with a third positioning post 183 and a fourth positioning post 184, respectively, and the middle part of the surface is provided with a second buckle 192.

The housing body 410 is provided with a first positioning hole configured to coordinate with the first positioning post 181, a second positioning hole configured to coordinate with the second positioning post 182, a third positioning hole configured to coordinate with the third positioning post 183, a fourth positioning hole configured to coordinate with the fourth positioning post 184, a first slot configured to coordinate with the first buckle 191, and a second slot configured to coordinate with the second buckle 192.

The gear fixing member is connected to the housing 4 through the connection between the positioning posts and the positioning holes as well as the connection between the buckles and the slots, ensuring that the assembling is accurate.

### Example 2

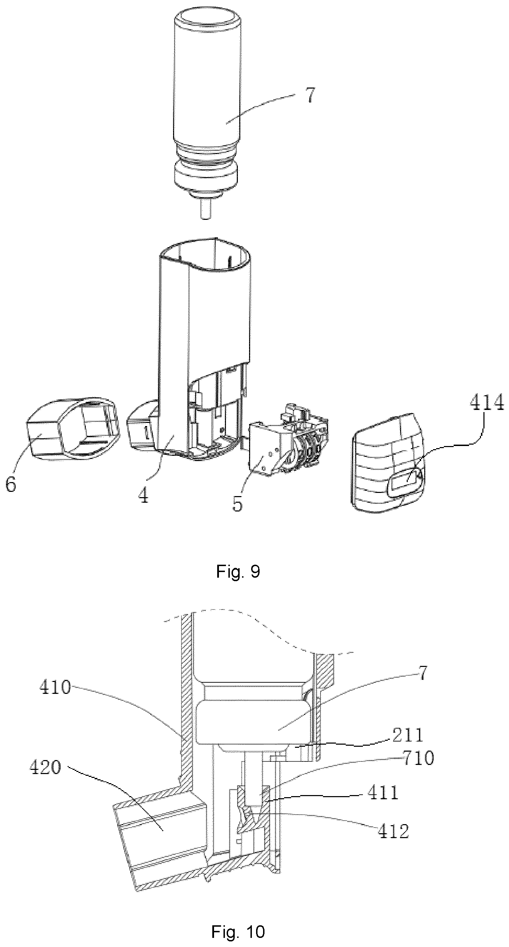

As shown in FIG. 9, the metered dose inhaler of the present disclosure comprises an aerosol canister 7 and the aerosol actuator of Example 1.

As shown in FIG. 10, at least part of the aerosol canister is installed in the housing body 410. A liquid outlet pipe 710 of the aerosol canister is installed in the liquid inlet passage 411. The edge of the cover body of the aerosol canister is in contact with the pressing part 211 of the driving element 2.

## Claims

1. An aerosol actuator, comprising a housing (4) and a counting unit (5);
wherein the housing comprises a housing body (410) and a nozzle (420), the housing body being configured to communicate with the nozzle; the housing body being configured to accommodate the counting unit;
the counting unit is arranged in the housing body, and the counting unit comprises a gear fixing member, a counting gear, a driving element (2), and an elastic member (3);
a cavity is formed inside the gear fixing member;
the gear fixing member comprises a cover plate part (110), the cover plate part comprising a cover plate part body (117) and a check pawl (113);
the counting gear is arranged in the cavity;
the check pawl is arranged on the cover plate part body, and the check pawl is configured to coordinate with a meshing tooth of the counting gear so as to limit a rotation direction of the counting gear;
the driving element is configured to move by an external force acting on it to thereby drive the counting gear to rotate;
the elastic member is configured to restore the driving element to its initial position when the external force is removed;
**characterized in that**,
a surface of the counting gear that is close to the cover plate part body is provided with a limiting recess (1512);
the check pawl is provided with a limiting protrusion (114);
the limiting protrusion is configured to coordinate with the limiting recess so as to limit a rotation direction of the counting gear.

2. The aerosol actuator according to claim 1, wherein each of numerals displayed on the counting gear corresponds to one of the meshing teeth; and each of the meshing teeth corresponds to one of the limiting recesses.

3. The aerosol actuator according to claim 1, wherein the counting unit comprises a linkage gear (161, 162); the number of the counting gears is at least two; two adjacent ones of the counting gears are linked through the linkage gear;
the gear fixing member further comprises a bracket part (120) configured to coordinate with the cover plate part; the bracket part comprises a bracket part body (133) and a bracket part stopper;
the counting gears comprise a lowest digit-position counting gear (151) and a highest digit-position counting gear (153); the lowest digit-position counting gear is close to the cover plate part body, and the highest digit-position counting gear is close to the bracket part body;
the lowest digit-position counting gear is provided with a limiting recess; the check pawl is configured to coordinate with a meshing tooth of the lowest digit-position counting gear to thereby limit a rotation direction of the lowest digit-position counting gear;
the bracket part stopper is arranged on the bracket part body;
a surface of the highest digit-position counting gear that is close to the bracket part body is provided with a stopping part (1531); the stopping part coordinates with the bracket part stopper so as to inhibit the highest digit-position counting gear from rotating in a counting direction when the highest digit-position counting gear counts down to 0.

4. The aerosol actuator according to claim 3, wherein the linkage gear is arranged between two adjacent ones of the counting gears; one side of the lowest digit-position counting gear that is close to the linkage gear is provided with a transmission tooth (1513), and one side of the lowest digit-position counting gear that is close to the cover plate part body is provided with a meshing tooth (1511); both sides of another low digit-position counting gear are provided with a transmission tooth and a meshing tooth, respectively; one side of the highest digit-position counting gear that is close to the linkage gear is provided with a meshing tooth;
the linkage gear is configured to be capable of engaging with the meshing tooth of one of two counting gears adjacent to it that is a high digit-position counting gear; the linkage gear and the counting gear are configured to function such that when a low digit-position counting gear counts down to 0 and then counts again, a transmission tooth of the low digit-position counting gear drives the linkage gear to rotate, and the linkage gear drives the high digit-position counting gear to rotate and count once.

5. The aerosol actuator according to claim 3, wherein the gear fixing member comprises a first fixing shaft (171) and a second fixing shaft (172); the first fixing shaft and the second fixing shaft are arranged in the cavity, the first fixing shaft is positioned higher than the second fixing shaft; the first fixing shaft is configured to fix the counting gear, and the second fixing shaft is configured to fix the linkage gear.

6. The aerosol actuator according to claim 1, wherein the driving element comprises a main body part (210) and a driving pawl (220);
the main body part is provided with a pressing part (211); the pressing part is configured to receive an external force to cause the main body part to move;
the driving pawl is connected to the main body part and configured to drive, through a meshing tooth, the counting gear to rotate;
the driving pawl is provided with a deflection bending part (221); the deflection bending part is configured such that when the counting gear rotates normally, the deflection bending part does not affect the driving pawl in driving the counting gear to rotate, and when the counting gear is locked and an external force acts on the pressing part, the deflection bending part elastically deforms and causes the driving element to move.

7. The aerosol actuator according to claim 6, wherein the driving element further comprises a connection part (230), and the gear fixing member is provided with a fixing part (116);
the elastic member is sleeved around the connection part, one end of the elastic member abuts against the main body part, and the other end of the elastic member abuts against the fixing part;
the fixing part is provided with a through hole, and at least part of the connection part penetrates the through hole.

8. The aerosol actuator according to claim 7, wherein the main body part is provided with a first normal limiting structure (212) and a first transverse limiting structure (213), and the gear fixing member is provided with a second normal limiting structure (115) and a second transverse limiting structure (132);
the first normal limiting structure coordinates with the second normal limiting structure to thereby limit a normal movement path of the driving element; the first transverse limiting structure coordinates with the second transverse limiting structure to thereby limit a transverse movement path of the driving element;
a lower part of the connection part is provided with a longitudinal limiting structure (231) configured to limit a longitudinal displacement of the driving element.

9. The aerosol actuator according to claim 1, wherein the aerosol actuator further comprises a dust cover (6);
a liquid inlet passage (411) is arranged in the housing body and provided with a spray hole (412); the liquid inlet passage is configured to communicate with the nozzle through the spray hole;
the dust cover is sleeved around the nozzle.

10. A metered dose inhaler, comprising an aerosol canister (7) and the aerosol actuator according to claim 9;
wherein at least part of the aerosol canister is configured to be accommodated in the housing body, and the liquid inlet passage is configured to communicate with a liquid outlet pipe (710) of the aerosol canister.

## Patentansprüche

1. Aerosolbetätigungsvorrichtung, die ein Gehäuse (4) und eine Zähleinheit (5) umfasst;
wobei das Gehäuse einen Gehäusekörper (410) und eine Düse (420) umfasst, wobei der Gehäusekörper dazu konfiguriert ist, mit der Düse zu kommunizieren; der Gehäusekörper dazu konfiguriert ist, die Zähleinheit aufzunehmen;
die Zähleinheit im Gehäusekörper angeordnet ist und die Zähleinheit ein Zahnradbefestigungselement, ein Zählrad, einem Antriebselement (2) und ein elastisches Element (3) umfasst;
ein Hohlraum im Inneren des Zahnradbefestigungselements gebildet ist;
das Zahnradbefestigungselement ein Abdeckplattenteil (110) umfasst, wobei das Abdeckplattenteil einen Abdeckplattenteilkörper (117) und eine Sperrklinke (113) umfasst;
das Zählwerk im Hohlraum angeordnet ist;
die Sperrklinke auf dem Abdeckplattenteilkörper angeordnet ist und die Sperrklinke dazu konfiguriert ist, mit einem Eingriffszahn des Zählrads zusammenzuwirken, um eine Drehrichtung des Zählrads zu begrenzen;
das Antriebselement dazu konfiguriert ist, sich durch eine äußere Kraft zu bewegen, die darauf wirkt, wodurch das Zählrad angetrieben wird, sich zu drehen;
das elastische Element dazu konfiguriert ist, das Antriebselement in seine Ausgangsposition zurückzuführen, wenn die äußere Kraft entfernt wird;
**dadurch gekennzeichnet, dass**
eine Oberfläche des Zählrads, die sich nahe dem Abdeckplattenteilkörper befindet, mit einer Begrenzungsvertiefung (1512) versehen ist;
die Sperrklinke mit einem Begrenzungsvorsprung (114) versehen ist;
der Begrenzungsvorsprung dazu konfiguriert ist, mit der Begrenzungsvertiefung zusammenzuwirken, um eine Drehrichtung des Zählrads zu begrenzen.

2. Aerosolbetätigungsvorrichtung nach Anspruch 1, wobei jede der auf dem Zählrad angezeigten Ziffern einem der Eingriffszähne entspricht; und jeder der Eingriffszähne einer der Begrenzungsvertiefungen entspricht.

3. Aerosolbetätigungsvorrichtung nach Anspruch 1, wobei die Zähleinheit ein Verbindungsrad (161, 162) umfasst; die Anzahl der Zählräder mindestens zwei beträgt; zwei benachbarte Zählräder durch das Verbindungsrad miteinander verbunden sind;
das Zahnradbefestigungselement ferner ein Halterungsteil (120) umfasst, das dazu konfiguriert ist, mit dem Abdeckplattenteil zusammenzuwirken; das Halterungsteil einen Halterungsteilkörper (133) und einen Halterungsteilanschlag umfasst;
die Zählräder ein Zählrad für die niedrigste Ziffernposition (151) und ein Zählrad für die höchste Ziffernposition (153) umfassen; das Zählrad für die niedrigste Ziffernposition sich nahe dem Abdeckplattenteilkörper befindet, und das Zählrad für die höchste Ziffernposition sich nahe dem Halterungsteilkörper befindet;
das Zählrad für die niedrigste Ziffernposition mit einer Begrenzungsvertiefung versehen ist; die Sperrklinke dazu konfiguriert ist, mit einem Eingriffszahn des Zählrads für die niedrigste Ziffernposition zusammenzuwirken, um dadurch eine Drehrichtung des Zählrads für die niedrigste Ziffernposition zu begrenzen;
der Halterungsteilanschlag am Halterungsteilkörper angeordnet ist;
eine Oberfläche des Zählrads für die höchste Ziffernposition, die sich nahe dem Halterungsteilkörper befindet, mit einem Anschlagteil (1531) versehen ist; das Anschlagteil mit dem Halterungsteilanschlag zusammenwirkt, um zu verhindern, dass sich das Zählrad für die höchste Ziffernposition in einer Zählrichtung dreht, wenn das Zählrad für die höchste Ziffernposition auf 0 herunterzählt.

4. Aerosolbetätigungsvorrichtung nach Anspruch 3, wobei das Verbindungsrad zwischen zwei benachbarten Zählrädern angeordnet ist; eine Seite des Zählrads für die niedrigste Ziffernposition, die sich nahe dem Verbindungsrad befindet, mit einem Übertragungszahn (1513) versehen ist, und eine Seite des Zählrads für die niedrigste Ziffernposition, die sich nahe dem Abdeckplattenteilkörpers befindet, mit einem Eingriffszahn (1511) versehen ist; beide Seiten eines weiteren Zählrads für eine niedrige Ziffernposition jeweils mit einem Übertragungszahn und einem Eingriffszahn versehen sind; eine Seite des Zählrads für die höchste Ziffernposition, die sich nahe dem Verbindungsrad befindet, ist mit einem Eingriffszahn versehen ist;
das Verbindungsrad dazu konfiguriert ist, im Stande zu sein, mit dem Eingriffszahn eines der beiden benachbarten Zählräder einzugreifen, das ein Zählrad für eine hohe Ziffernposition ist; das Verbindungsrad und das Zählrad dazu konfiguriert sind, so zu fungieren, dass wenn ein Zählrad für eine niedrigere Ziffernposition bis 0 herunterzählt und dann wieder zählt, ein Übertragungszahn des Zählrads für eine niedrige Ziffernposition das Verbindungsrad antreibt, sich zu drehen, und das Verbindungsrad das Zählrad für eine hohe Ziffernposition antreibt, sich zu drehen und einmal zu zählen.

5. Aerosolbetätigungsvorrichtung nach Anspruch 3, wobei das Zahnradbefestigungselement eine erste Befestigungswelle (171) und eine zweite Befestigungswelle (172) umfasst; die erste Befestigungswelle und die zweite Befestigungswelle in dem Hohlraum angeordnet sind, wobei die erste Befestigungswelle höher als die zweite Befestigungswelle positioniert ist; die erste Befestigungswelle dazu konfiguriert ist, das Zählrad zu befestigen, und die zweite Befestigungswelle dazu konfiguriert ist, das Verbindungsrad zu befestigen.

6. Aerosolbetätigungsvorrichtung nach Anspruch 1, wobei das Antriebselement einen Hauptkörperteil (210) und eine Antriebsklinke (220) umfasst;
das Hauptkörperteil mit einem Pressteil (211) versehen ist; das Pressteil dazu konfiguriert ist, eine äußere Kraft aufzunehmen, um das Hauptkörperteil zu veranlassen, sich zu bewegen;
die Antriebsklinke mit dem Hauptkörperteil verbunden und dazu konfiguriert ist, über einen Eingriffszahn das Zählrad anzutreiben, sich zu drehen;
die Antriebsklinke mit einem Ablenkungsbiegeteil (221) versehen ist; das Ablenkungsbiegeteil so konfiguriert ist, dass wenn sich das Zählrad normal dreht, das Ablenkungsbiegeteil die Antriebsklinke nicht darin beeinträchtigt, das Zählrad anzutreiben, sich zu drehen, und wenn das Zählrad verriegelt ist und eine äußere Kraft auf das Pressteil wirkt, das Ablenkungsbiegeteil sich elastisch verformt und das Antriebselement veranlasst, sich zu bewegen.

7. Aerosolbetätigungsvorrichtung nach Anspruch 6, wobei das Antriebselement ferner ein Verbindungsteil (230) umfasst und das Zahnradbefestigungselement mit einem Befestigungsteil (116) versehen ist;
das elastische Element um das Verbindungsteil herum angebracht ist, ein Ende des elastischen Elements am Hauptkörperteil anliegt, und das andere Ende des elastischen Elements am Befestigungsteil anliegt;
das Befestigungsteil mit einer Durchgangsbohrung versehen ist, und mindestens ein Teil des Verbindungsteils die Durchgangsbohrung durchdringt.

8. Aerosolbetätigungsvorrichtung nach Anspruch 7, wobei das Hauptkörperteil mit einer ersten normalen Begrenzungsstruktur (212) und einer ersten querverlaufenden Begrenzungsstruktur (213) versehen ist und das Zahnradbefestigungselement mit einer zweiten normalen Begrenzungsstruktur (115) und einer zweiten querverlaufenden Begrenzungsstruktur (132) versehen ist;
die erste normale Begrenzungsstruktur mit der zweiten normalen Begrenzungsstruktur zusammenwirkt, um dadurch einen normalen Bewegungsweg des Antriebselements zu begrenzen; die erste querverlaufende Begrenzungsstruktur mit der zweiten querverlaufenden Begrenzungsstruktur zusammenwirkt, um dadurch einen querverlaufenden Bewegungsweg des Antriebselements zu begrenzen;
ein unterer Teil des Verbindungsteils mit einer Längsbegrenzungsstruktur (231) versehen ist, die dazu konfiguriert ist, eine Längsverschiebung des Antriebselements zu begrenzen.

9. Aerosolbetätigungsvorrichtung nach Anspruch 1, wobei die Aerosolbetätigungsvorrichtung ferner eine Staubschutzkappe (6) umfasst;
ein Flüssigkeitseinlasskanal (411) im Gehäusekörper angeordnet und mit einer Sprühöffnung (412) versehen ist; der Flüssigkeitseinlasskanal dazu konfiguriert ist, über die Sprühöffnung mit der Düse zu kommunizieren;
die Staubschutzkappe um die Düse herum angebracht ist.

10. Dosierinhalator, umfassend einen Aerosolbehälter (7) und die Aerosolbetätigungsvorrichtung nach Anspruch 9;
wobei zumindest ein Teil des Aerosolbehälters dazu konfiguriert ist, im Gehäusekörper untergebracht zu werden, und der Flüssigkeitseinlasskanal dazu konfiguriert ist, mit einem Flüssigkeitsauslassrohr (710) des Aerosolbehälters zu kommunizieren.

## Revendications

1. Actionneur d'aérosol, comprenant un boîtier (4) et une unité de comptage (5) ;
dans lequel le boîtier comprend un corps de boîtier (410) et une buse (420), le corps de boîtier étant configuré pour communiquer avec la buse ; le corps de boîtier étant configuré pour loger l'unité de comptage ;
l'unité de comptage est disposée dans le corps de boîtier et l'unité de comptage comprend un élément de fixation d'engrenage, un engrenage de comptage, un élément d'entraînement (2) et un élément élastique (3) ;
une cavité est formée à l'intérieur de l'élément de fixation d'engrenage ;
l'élément de fixation d'engrenage comprend une partie formant plaque de recouvrement (110), la partie formant plaque de recouvrement comprenant un corps de partie formant plaque de recouvrement (117) et un cliquet de retenue (113) ;
l'engrenage de comptage est disposé dans la cavité ;
le cliquet de retenue est disposé sur le corps de partie formant plaque de recouvrement et le cliquet de retenue est configuré pour se coordonner avec une dent d'engrènement de l'engrenage de comptage de façon à limiter un sens de rotation de l'engrenage de comptage ;
l'élément d'entraînement est configuré pour se déplacer sous l'effet d'une force externe agissant sur celui-ci pour ainsi entraîner une rotation de l'engrenage de comptage ;
l'élément élastique est configuré pour ramener l'élément d'entraînement à sa position initiale lorsque la force externe est supprimée ;
**caractérisé en ce qu'**
une surface de l'engrenage de comptage qui est proche du corps de partie formant plaque de recouvrement est munie d'un évidement de limitation (1512) ;
le cliquet de retenue est muni d'une protubérance de limitation (114) ;
la protubérance de limitation est configurée pour se coordonner avec l'évidement de limitation de façon à limiter un sens de rotation de l'engrenage de comptage.

2. Actionneur d'aérosol selon la revendication 1, dans lequel chacun des chiffres affichés sur l'engrenage de comptage correspond à l'une des dents d'engrènement ; et chacune des dents d'engrènement correspond à l'un des évidements de limitation.

3. Actionneur d'aérosol selon la revendication 1, dans lequel l'unité de comptage comprend un engrenage de liaison (161, 162) ; le nombre des engrenages de comptage est d'au moins deux ; deux engrenages de comptage adjacents des engrenages de comptage sont liés par le biais de l'engrenage de liaison ;
l'élément de fixation d'engrenage comprend en outre une partie de support (120) configurée pour se coordonner avec la partie formant plaque de recouvrement ; la partie de support comprend un corps de partie de support (133) et une butée de partie de support ;
les engrenages de comptage comprennent un engrenage de comptage de position de chiffre le plus bas (151) et un engrenage de comptage de position de chiffre le plus haut (153) ; l'engrenage de comptage de position de chiffre le plus bas est proche du corps de partie formant plaque de recouvrement et l'engrenage de comptage de position de chiffre le plus haut est proche du corps de partie de support ;
l'engrenage de comptage de position de chiffre le plus bas est muni d'un évidement de limitation ; le cliquet de retenue est configuré pour se coordonner avec une dent d'engrènement de l'engrenage de comptage de position de chiffre le plus bas pour ainsi limiter un sens de rotation de l'engrenage de comptage de position de chiffre le plus bas ;
la butée de partie support est disposée sur le corps de partie support ;
une surface de l'engrenage de comptage de position de chiffre le plus haut qui est proche du corps de partie de support est munie d'une partie de butée (1531) ; la partie de butée se coordonne avec la butée de partie de support de façon à empêcher l'engrenage de comptage de position de chiffre le plus haut de tourner dans un sens de comptage lorsque l'engrenage de comptage de position de chiffre le plus haut compte jusqu'à 0.

4. Actionneur d'aérosol selon la revendication 3, dans lequel l'engrenage de liaison est disposé entre deux engrenages de comptage adjacents des engrenages de comptage ; un côté de l'engrenage de comptage de position de chiffre le plus bas qui est proche de l'engrenage de liaison est muni d'une dent de transmission (1513) et un côté de l'engrenage de comptage de position de chiffre le plus bas qui est proche du corps de partie formant plaque de recouvrement est muni d'une dent d'engrènement (1511) ; les deux côtés d'un autre engrenage de comptage de position de chiffre bas sont munis d'une dent de transmission et d'une dent d'engrènement, respectivement ; un côté de l'engrenage de comptage de position de chiffre le plus haut qui est proche de l'engrenage de liaison est muni d'une dent d'engrènement ;
l'engrenage de liaison est configuré pour pouvoir se mettre en prise avec la dent d'engrènement de l'un des deux engrenages de comptage adjacents à celui-ci qui est un engrenage de comptage de position de chiffre haut ; l'engrenage de liaison et l'engrenage de comptage sont configurés pour fonctionner de telle sorte que lorsqu'un engrenage de comptage de position de chiffre bas décompte jusqu'à 0 puis compte à nouveau, une dent de transmission de l'engrenage de comptage de position de chiffre bas entraîne la rotation de l'engrenage de liaison et l'engrenage de liaison entraîne la rotation et le comptage une fois de l'engrenage de comptage de position de chiffre haut.

5. Actionneur d'aérosol selon la revendication 3, dans lequel l'élément de fixation d'engrenage comprend un premier arbre de fixation (171) et un second arbre de fixation (172) ; le premier arbre de fixation et le second arbre de fixation sont disposés dans la cavité, le premier arbre de fixation est positionné plus haut que le second arbre de fixation ; le premier arbre de fixation est configuré pour fixer l'engrenage de comptage et le second arbre de fixation est configuré pour fixer l'engrenage de liaison.

6. Actionneur d'aérosol selon la revendication 1, dans lequel l'élément d'entraînement comprend une partie formant corps principal (210) et un cliquet d'entraînement (220) ;
la partie formant corps principal est munie d'une partie de pression (211) ; la partie de pression est configurée pour recevoir une force externe pour amener la partie formant corps principal à se déplacer ;
le cliquet d'entraînement est relié à la partie formant corps principal et configuré pour entraîner, par le biais d'une dent d'engrènement, la rotation de l'engrenage de comptage ;
le cliquet d'entraînement est muni d'une partie de flexion par déviation (221) ; la partie de flexion par déviation est configurée de telle sorte que lorsque l'engrenage de comptage tourne normalement, la partie de flexion par déviation n'affecte pas le cliquet d'entraînement dans l'entraînement de la rotation de l'engrenage de comptage et lorsque l'engrenage de comptage est bloqué et qu'une force externe agit sur la partie de pression, la partie de flexion par déviation se déforme élastiquement et provoque le déplacement de l'élément d'entraînement.

7. Actionneur d'aérosol selon la revendication 6, dans lequel l'élément d'entraînement comprend en outre une partie de connexion (230) et l'élément de fixation d'engrenage est muni d'une partie de fixation (116) ;
l'élément élastique est gainé autour de la partie de connexion, une extrémité de l'élément élastique bute contre la partie formant corps principal et l'autre extrémité de l'élément élastique bute contre la partie de fixation ;
la partie de fixation est munie d'un trou traversant et au moins une partie de la partie de connexion pénètre dans le trou traversant.

8. Actionneur d'aérosol selon la revendication 7, dans lequel la partie formant corps principal est munie d'une première structure de limitation perpendiculaire (212) et d'une première structure de limitation transversale (213) et l'élément de fixation d'engrenage est muni d'une seconde structure de limitation perpendiculaire (115) et d'une seconde structure de limitation transversale (132) ;
la première structure de limitation perpendiculaire se coordonne avec la seconde structure de limitation perpendiculaire pour ainsi limiter une trajectoire de mouvement perpendiculaire de l'élément d'entraînement ; la première structure de limitation transversale se coordonne avec la seconde structure de limitation transversale pour ainsi limiter une trajectoire de mouvement transversale de l'élément d'entraînement ;
une partie inférieure de la partie de connexion est munie d'une structure de limitation longitudinale (231) configurée pour limiter un déplacement longitudinal de l'élément d'entraînement.

9. Actionneur d'aérosol selon la revendication 1, dans lequel l'actionneur d'aérosol comprend en outre un couvercle anti-poussière (6) ;
un passage d'entrée de liquide (411) est disposé dans le corps de boîtier et muni d'un trou de pulvérisation (412) ; le passage d'entrée de liquide est configuré pour communiquer avec la buse à travers le trou de pulvérisation ;
le couvercle anti-poussière est gainé autour de la buse.

10. Inhalateur-doseur comprenant une cartouche d'aérosol (7) et un actionneur d'aérosol selon la revendication 9 ;
dans lequel au moins une partie de la cartouche d'aérosol est configurée pour être logée dans le corps de boîtier et le passage d'entrée de liquide est configuré pour communiquer avec un tuyau de sortie de liquide (710) de la cartouche d'aérosol.
